# EUROPEAN PATENT APPLICATION

(11) **EP 4 364 596 A1**
(43) Date of publication of application: **08.05.2024**
(21) Application number: 21955084.5
(22) Date of filing: 27.08.2021
(51) Int. Cl.: A24F 40/50, A24F 40/60

(54) **INFORMATION PROCESSING METHOD, AEROSOL GENERATION SYSTEM, AND PROGRAM**

(71) Applicant: Japan Tobacco, Inc., Tokyo, 105-6927 (JP)
(72) Inventor: SENJU, Masatoshi, Tokyo 130-8603 (JP); SUGANO, Yuka, Tokyo 130-8603 (JP); SERITA, Kazutoshi, Tokyo 130-8603 (JP)
(74) Representative: Hoffmann Eitle
(86) International application number: PCT/JP2021/031510
(87) International publication number: WO 2023/026474

(57) **Abstract**

[Problem] To provide a biometric authentication mechanism that allows for a further increase in user convenience. [Solution] An information processing method comprising: acquiring a predetermined request for requesting an aspiration apparatus, which generates an aerosol to be aspirated by a user, to execute a predetermined process; and determining, depending on whether or not a predetermined condition is met, whether or not to perform biometric authentication based on biometric information of the user before execution of the predetermined process.

## Description

### Technical Field

The present invention relates to an information processing method, an aerosol generation system, and a program.

### Background Art

Inhaler devices, such as e-cigarettes and nebulizers, for generating a substance to be inhaled by a user are widespread. For example, an inhaler device generates an aerosol to which a flavor component is imparted, by using a substrate including an aerosol source for generating the aerosol, a flavor source for imparting the flavor component to the generated aerosol, and the like. The user inhales the aerosol to which the flavor component is imparted, which is generated by the inhaler device, to taste the flavor. The action of the user inhaling the aerosol is hereinafter also referred to as a puff or a puff action.

An inhaler device provided with a biometric sensor for detecting biometric information has recently been under consideration. For example, Patent Literature 1 below discloses a technique for authenticating a user on the basis of biometric information detected by a biometric sensor mounted on an inhaler device.

### Citation List

### Patent Literature

Patent Literature 1: US 20190175846 A1

### Summary of Invention

### Technical Problem

However, the technique described in Patent Literature 1 above has not been developed for a long time and has room for improvement from various viewpoints.

The present invention has been made in view of the above problem, and it is an object of the present invention to provide a biometric authentication mechanism that allows a further increase in user convenience.

### Solution to Problem

In order to solve the above problem, an aspect of the present invention provides an information processing method including acquiring a predetermined request for requesting an inhaler device to execute a predetermined process, the inhaler device being configured to generate an aerosol to be inhaled by a user; and determining whether to perform biometric authentication based on biometric information of the user before the predetermined process is executed, in accordance with whether a predetermined condition is satisfied.

The determining of whether to perform biometric authentication may include determining not to perform the biometric authentication before the predetermined process is executed, when the predetermined condition is satisfied; and determining to perform the biometric authentication before the predetermined process is executed, when the predetermined condition is not satisfied.

The information processing method may further include controlling the inhaler device to execute the predetermined process when the predetermined condition is satisfied.

The information processing method may further include, when the predetermined condition is not satisfied, acquiring the biometric information of the user; performing the biometric authentication, based on the acquired biometric information; and controlling the inhaler device to execute the predetermined process in response to the biometric authentication being successful.

The predetermined condition may include a condition that the inhaler device has executed the predetermined process within a first predetermined time before the predetermined request is acquired.

The first predetermined time may be set in accordance with a position of the user.

The first predetermined time may be set in accordance with a situation around the user.

The first predetermined time may be set in accordance with a time period.

The predetermined condition may include a condition that the biometric authentication based on the biometric information acquired within a second predetermined time before the predetermined request is acquired has succeeded.

The predetermined process may be a process of permitting execution of a process of generating the aerosol.

The biometric authentication may be performed based on a plurality of types of the biometric information.

The predetermined request may be acquired by the inhaler device.

The predetermined request may be acquired by a terminal device associated with the inhaler device in advance.

The biometric information may be acquired by a wearable terminal worn by the user.

The biometric authentication may be performed by the inhaler device.

The biometric authentication may be performed by a terminal device associated with the inhaler device in advance.

The biometric authentication may be performed by a server located on the Internet.

In order to solve the above problem, another aspect of the present invention provides an aerosol generation system including a request acquirer configured to acquire a predetermined request for requesting an inhaler device to execute a predetermined process, the inhaler device being configured to generate an aerosol to be inhaled by a user; and a device controller configured to determine whether to perform biometric authentication based on biometric information of the user before the predetermined process is executed, in accordance with whether a predetermined condition is satisfied.

The inhaler device may be configured to generate the aerosol by using a substrate including at least one of an aerosol source from which the aerosol is generated or a flavor source from which a flavor to be imparted to the aerosol is generated, and the aerosol generation system may include the substrate.

In order to solve the above problem, another aspect of the present invention provides a program for causing a computer to execute, in response to acquisition of a predetermined request for requesting an inhaler device to execute a predetermined process, the inhaler device being configured to generate an aerosol to be inhaled by a user, determining whether to perform biometric authentication based on biometric information of the user before the predetermined process is executed, in accordance with whether a predetermined condition is satisfied.

### Advantageous Effects of Invention

As described above, according to the present invention, there is provided a biometric authentication mechanism that allows a further increase in user convenience.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a block diagram illustrating a configuration example of a system according to a first embodiment.
[Fig. 4] Fig. 4 is a sequence diagram illustrating an example of a process flow executed in the system according to the first embodiment.
[Fig. 5] Fig. 5 is a block diagram illustrating a configuration example of a system according to a second embodiment.
[Fig. 6] Fig. 6 is a sequence diagram illustrating an example of a process flow executed in the system according to the second embodiment.
[Fig. 7] Fig. 7 is a block diagram illustrating a configuration example of a system according to a third embodiment.
[Fig. 8] Fig. 8 is a sequence diagram illustrating an example of a process flow executed in the system according to the third embodiment.
[Fig. 9] Fig. 9 is a block diagram illustrating a configuration example of a system according to a fourth embodiment.
[Fig. 10] Fig. 10 is a sequence diagram illustrating an example of a process flow executed in the system according to the fourth embodiment.
[Fig. 11] Fig. 11 is a block diagram illustrating an example of a hardware configuration of an information processing device according to the first to fourth embodiments.

### Description of Embodiments

Preferred embodiments of the present invention will be described in detail hereinafter with reference to the accompanying drawings. In the specification and the drawings, structural elements having substantially the same functional configuration are denoted by the same reference numerals, and redundant description thereof will be omitted.

### <1. Configuration example of inhaler device>

An inhaler device generates material to be inhaled by a user. In the example described below, the material generated by the inhaler device is an aerosol. Alternatively, the material generated by the inhaler device may be gas.

### (1) First configuration example

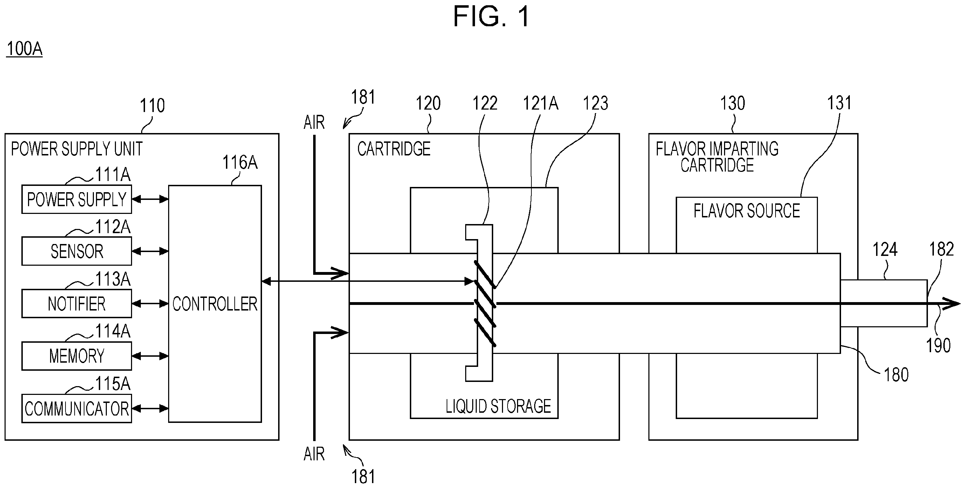

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a condenser microphone, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) and a microprocessor, for example.

The liquid storage 123 stores an aerosol source. The aerosol source is atomized to generate an aerosol. The aerosol source is a liquid such as polyhydric alcohol and water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

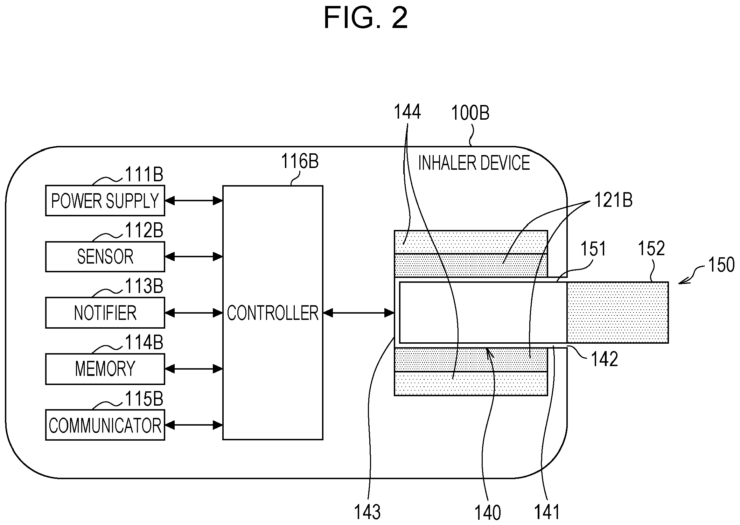

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 accommodates the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining a flow path of air to be supplied to the stick substrate 150. An air inlet hole, which is an inlet of air to the flow path, is disposed in the bottom 143, for example. An air outlet hole, which is an outlet of air from the flow path, is the opening 142.

The stick substrate 150 includes a substrate 151 and an inhalation port 152. The substrate 151 includes an aerosol source. In the present configuration example, the aerosol source is not limited to a liquid, and may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 from the air inlet hole (not illustrated), and the air and an aerosol generated from the substrate 151 reach inside the mouth of the user.

The heater 121B has a configuration similar to that of the heater 121A according to the first configuration example. In the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as the air inlet hole to the internal space 141. In this case, a mixture fluid of the air and an aerosol generated by the heater 121A flows into the internal space 141, mixes further with an aerosol generated by the heater 121B, and then reaches the oral cavity of the user.

### <2. Technical features>

### <2.1. First embodiment>

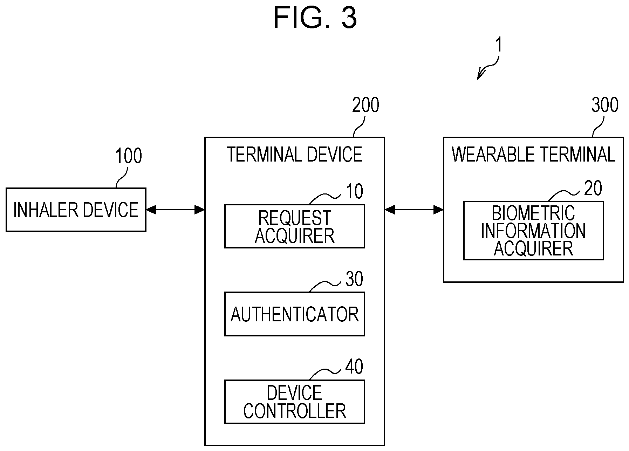

Fig. 3 is a block diagram illustrating a configuration example of a system 1 according to the first embodiment. As illustrated in Fig. 3, the system 1 according to the present embodiment includes an inhaler device 100, a terminal device 200, and a wearable terminal 300 as physical structural elements. The inhaler device 100 and the terminal device 200 can communicate with each other, and the terminal device 200 and the wearable terminal 300 can communicate with each other.

The system 1 according to the present embodiment includes a request acquirer 10, a biometric information acquirer 20, an authenticator 30, and a device controller 40 as logical structural elements. As illustrated in Fig. 3, in the present embodiment, the terminal device 200 includes the request acquirer 10, the authenticator 30, and the device controller 40, and the wearable terminal 300 includes the biometric information acquirer 20.

### (1) Physical structural elements

The inhaler device 100 may have any configuration example among the first configuration example and the second configuration example described above. The inhaler device 100 generates an aerosol by using a substrate including at least one of an aerosol source from which the aerosol is generated or a flavor source from which a flavor to be imparted to the aerosol is generated. The cartridge 120, the flavor imparting cartridge 130, and the stick substrate 150 described above are examples of the substrate in the present embodiment. In the first configuration example, since an aerosol can be generated by combining the power supply unit 110, the cartridge 120, and the flavor imparting cartridge 130, a combination of the power supply unit 110, the cartridge 120, and the flavor imparting cartridge 130 may be regarded as one aerosol generation system. Likewise, in the second configuration example, since an aerosol can be generated by combining the inhaler device 100 and the stick substrate 150, a combination of the inhaler device 100 and the stick substrate 150 may be regarded as one aerosol generation system. In addition, the entire system 1 may be regarded as an aerosol generation system.

The terminal device 200 is a device used by the user of the inhaler device 100. The terminal device 200 is, for example, any information processing device such as a smartphone or a tablet terminal. The terminal device 200 is associated with the inhaler device 100 in advance. For example, the terminal device 200 is paired with the inhaler device 100 in advance, and controls the operation of the inhaler device 100 via wireless communication or acquires and stores the operation history of the inhaler device 100. Depending on the communication standard for wireless communication between the inhaler device 100 and the terminal device 200, the terminal device 200 does not necessarily have to be associated with the inhaler device 100 in advance.

The wearable terminal 300 is a device to be worn by the user of the inhaler device 100. The wearable terminal 300 may be of any type such as a wristwatch type, a necklace type, or a contact lens type. The wearable terminal 300 is associated with the terminal device 200 in advance. For example, the terminal device 200 is paired with the wearable terminal 300 in advance, and controls the operation of the wearable terminal 300 via wireless communication or acquires and stores the operation history of the wearable terminal 300. The wearable terminal 300 may further be associated with the inhaler device 100 in advance. As an example, the wearable terminal 300 may be directly paired with the inhaler device 100. As another example, the wearable terminal 300 may be associated with the inhaler device 100 indirectly through the terminal device 200 by being paired with the terminal device 200 paired with the inhaler device 100. Depending on the communication standard for wireless communication, the wearable terminal 300 does not necessarily have to be associated with the terminal device 200 or the inhaler device 100 in advance.

### (2) Logical structural elements

### - Request acquirer 10

The request acquirer 10 acquires a predetermined request for requesting the inhaler device 100 to execute a predetermined process. When acquiring the predetermined request, the request acquirer 10 transmits information indicating that the predetermined request has been acquired to the device controller 40. At this time, the request acquirer 10 may transmit information indicating a timing (for example, time) at which the predetermined request is acquired to the device controller 40 together with the information indicating that the predetermined request has been acquired. Alternatively, the device controller 40 may handle a timing at which the information indicating that the predetermined request has been acquired is received as the timing at which the predetermined request is acquired.

An example of the predetermined process is a process of permitting execution of a process of generating an aerosol. The inhaler device 100 prohibits or permits generation of an aerosol. The inhaler device 100 can generate an aerosol only in a situation where the generation of an aerosol is permitted. Prohibiting the inhaler device 100 from generating an aerosol is also referred to as locking the inhaler device 100. On the other hand, permitting the inhaler device 100 to generate an aerosol is also referred to as unlocking. That is, the predetermined request may be an unlock request. In the following, the predetermined process is also referred to as unlocking, and the predetermined request is also referred to as an unlock request.

The request acquirer 10 according to the present embodiment is mounted on the terminal device 200. Accordingly, an unlock request is acquired by the terminal device 200. For example, the unlock request is acquired as a user input to an input device such as a button or a touch panel of the terminal device 200. Alternatively, the unlock request may be acquired as a voice input or a gesture input.

### - Biometric information acquirer 20

The biometric information acquirer 20 acquires biometric information of the user. The biometric information is information indicating a physical feature or a behavioral feature of a living body. Examples of the information indicating a physical feature include a fingerprint, a heart rate, a pulse, an iris, a face, a blood vessel, a body temperature, and a voiceprint. Examples of the information indicating a behavioral feature include handwriting and gait. The biometric information acquirer 20 acquires at least one of these pieces of biometric information.

The biometric information acquirer 20 acquires biometric information in response to a request from the device controller 40. When acquiring the biometric information, the biometric information acquirer 20 transmits the acquired biometric information to the authenticator 30.

The biometric information acquirer 20 according to the present embodiment is mounted on the wearable terminal 300. The biometric information acquirer 20 acquires biometric information by a biometric sensor included in the wearable terminal 300. Examples of the biometric sensor include a fingerprint sensor, a heart rate sensor, a pulse sensor, a blood pressure sensor, a respiration sensor, an electromyography sensor, a temperature sensor, a gyro sensor, an acceleration sensor, a camera, and a microphone.

### - Authenticator 30

The authenticator 30 performs biometric authentication, based on the biometric information acquired by the biometric information acquirer 20. The authenticator 30 compares the biometric information acquired by the biometric information acquirer 20 with biometric information registered in the past as the biometric information of the user of the inhaler device 100 to perform authentication. For example, the authenticator 30 determines that the authentication is successful when the degree of matching between the two pieces of biometric information is equal to or greater than a threshold, and determines that the authentication fails otherwise.

The authenticator 30 may perform biometric authentication, based on a plurality of types of biometric information. For example, the authenticator 30 performs biometric authentication by using different types of biometric information such as a heart rate and a fingerprint. This configuration enables an increase in authentication accuracy.

The authenticator 30 performs biometric authentication in response to receiving biometric information from the biometric information acquirer 20. Then, the authenticator 30 transmits information indicating the result of the biometric authentication to the device controller 40. The information indicating the result of the biometric authentication is information indicating the success or failure of the biometric authentication.

### - Device controller 40

The device controller 40 controls the operation of the inhaler device 100. Specifically, when the unlock request is acquired, the device controller 40 permits or prohibits the execution of the process of generating an aerosol by the inhaler device 100. When the generation of an aerosol is permitted, the inhaler device 100 can generate an aerosol in accordance with a user operation. For example, when the generation of an aerosol is permitted, the inhaler device 100 supplies power to the means for atomizing the aerosol source in accordance with a user operation. On the other hand, when the generation of an aerosol is prohibited, the inhaler device 100 does not generate an aerosol. For example, when the generation of an aerosol is prohibited, the inhaler device 100 does not supply power to the means for atomizing the aerosol source according to a user operation.

The device controller 40 controls the operation of the inhaler device 100 in accordance with the result of the biometric authentication. For example, the device controller 40 permits the generation of an aerosol when the biometric authentication is successful, and prohibits the generation of an aerosol when the biometric authentication fails.

Further, the device controller 40 may permit or prohibit the reception of a user operation on the inhaler device 100 in accordance with the result of the biometric authentication. When the reception of a user operation is permitted, the inhaler device 100 receives the user operation. For example, when the reception of a user operation is permitted, the inhaler device 100 receives various user operations on the inhaler device 100, such as the user starting inhaling and the user inputting predetermined information. On the other hand, when the reception of a user operation is prohibited, the inhaler device 100 does not receive the user operation. For example, when the reception of a user operation is prohibited, the inhaler device 100 does not receive various user operations on the inhaler device 100, such as the user starting inhaling and the user inputting predetermined information. When the reception of a user operation is prohibited, the inhaler device 100 may receive none of the user operations on the inhaler device 100 or may fail to receive some user operations related to the generation of an aerosol. Further, prohibiting the reception of a user operation on the inhaler device 100 may also be referred to as locking the inhaler device 100.

Note that the device controller 40 determines whether to perform biometric authentication based on the biometric information of the user before unlocking is performed, in accordance with whether a predetermined condition is satisfied. That is, the device controller 40 determines whether to perform unlocking when biometric authentication is performed and the authentication is successful or to perform unlocking without performing biometric authentication. In the present embodiment, it is possible to perform unlocking without performing biometric authentication. Thus, unlocking can be performed earlier than when unlocking is performed after biometric authentication is performed without fail. Accordingly, a time lag from when the user inputs an unlock request to when unlocking is actually performed can be reduced. In this way, the usability of the inhaler device 100 can be improved.

When the predetermined condition is satisfied, the device controller 40 determines not to perform biometric authentication before unlocking is performed. That is, when the predetermined condition is satisfied, the device controller 40 controls the inhaler device 100 such that unlocking is performed. This configuration allows unlocking without performing biometric authentication when the predetermined condition is satisfied. Therefore, the usability of the inhaler device 100 can be improved.

On the other hand, when the predetermined condition is not satisfied, the device controller 40 determines to perform biometric authentication before unlocking is performed. That is, when the predetermined condition is not satisfied, the device controller 40 requests the biometric information acquirer 20 to acquire biometric information. When receiving the request, the biometric information acquirer 20 acquires biometric information and transmits the biometric information to the authenticator 30. The authenticator 30 performs biometric authentication, based on the received biometric information. Then, the device controller 40 controls the inhaler device 100 such that unlocking is performed when the biometric authentication is successful. On the other hand, when the biometric authentication fails, the device controller 40 does not unlock the inhaler device 100. This configuration allows unlocking after biometric authentication is performed when the predetermined condition is not satisfied. Therefore, the inhaler device 100 can be prevented from being used by a person other than the user, and accordingly security can be improved.

The predetermined condition may include a condition that the inhaler device 100 has been unlocked within a first predetermined time before an unlock request is acquired. That is, the device controller 40 may determine to perform unlocking without performing biometric authentication if the next unlock request is acquired within the first predetermined time after unlocking is performed previously. If the elapsed time from the previous unlocking is short, it is considered that the same user continues to use the inhaler device 100. Since it is considered that the biometric authentication is successful at the previous unlocking time, security can be ensured even if no biometric authentication is performed at the next unlocking time. Therefore, this configuration makes it possible to improve usability while maintaining security.

The first predetermined time may be set in accordance with the position of the user. As an example, when the user is located in a place where only the user is present, such as the user's room, the device controller 40 sets the first predetermined time to be long. This is because only the user uses the inhaler device 100. As another example, when the user is located in a place where a person other than the user is present, such as a public space, the device controller 40 sets the first predetermined time to be short. This is because another person may use the inhaler device 100 by mistake. Further, when the user is located in a place where a child or the like, who is prohibited from smoking, is present, such as the user's house, the device controller 40 may set the first predetermined time to be short. This is because a child or the like, who is prohibited from smoking, may use the inhaler device 100 by mistake. This configuration increases or decreases the first predetermined time in accordance with the possibility of the inhaler device 100 being erroneously used by another person or a child, who is prohibited from smoking. As a result, the balance between security and usability can be optimized.

The first predetermined time may be set in accordance with the situation around the user. As an example, the device controller 40 sets the first predetermined time to be long in a situation in which no other person is present around the user. This is because only the user uses the inhaler device 100. As another example, the device controller 40 sets the first predetermined time to be short in a situation in which another person is present around the user. This is because another person may use the inhaler device 100 by mistake. This configuration increases or decreases the first predetermined time in accordance with the possibility of the inhaler device 100 being erroneously used by another person. As a result, the balance between security and usability can be optimized.

The first predetermined time may be set in accordance with a time period. As an example, the device controller 40 sets the first predetermined time to be long in a time period during which the user is expected to be in the user's room, such as the time period from night to morning. This is because only the user uses the inhaler device 100. As another example, the device controller 40 sets the first predetermined time to be short in a time period during which the user is expected to be away from home such as daytime. This is because another person may use the inhaler device 100 by mistake. The device controller 40 may set the first predetermined time to be short in the time period from night to morning or the like during which the user is expected to be located in a place such as the user's house where a child or the like, who is prohibited from smoking, is present. This is because a child or the like, who is prohibited from smoking, may use the inhaler device 100 by mistake. On the other hand, the device controller 40 may set the first predetermined time to be long in a time period during which the user is expected to be away from home such as daytime. This is because the inhaler device 100 is not expected to be used by a child or the like, who is prohibited from smoking. This configuration increases or decreases the first predetermined time in accordance with the possibility of the inhaler device 100 being erroneously used by another person or a child, who is prohibited from smoking. As a result, the balance between security and usability can be optimized.

### (3) Process flow

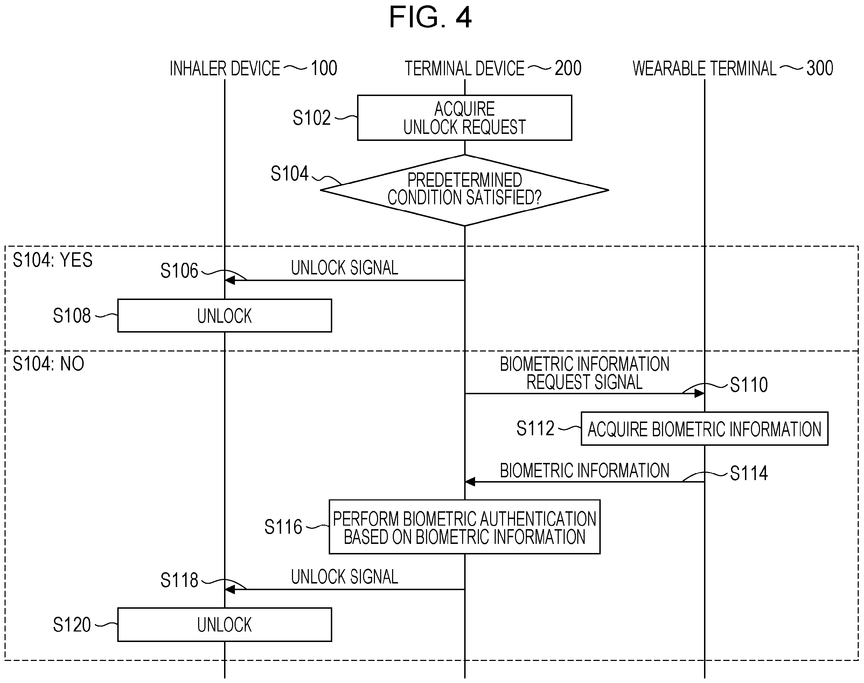

Fig. 4 is a sequence diagram illustrating an example of a process flow executed in the system 1 according to the present embodiment. This sequence involves the inhaler device 100, the terminal device 200, and the wearable terminal 300.

As illustrated in Fig. 4, first, the terminal device 200 acquires an unlock request (step S102).

Then, the terminal device 200 determines whether a predetermined condition is satisfied (step S104). For example, the terminal device 200 determines whether an unlock request has been acquired within the first predetermined time after unlocking was performed previously.

If it is determined that the predetermined condition is satisfied (step S104: YES), the terminal device 200 transmits an unlock request signal to the inhaler device 100 (step S106).

When receiving the unlock request signal, the inhaler device 100 is unlocked (step S108). Thereafter, the inhaler device 100 generates an aerosol in accordance with a user operation.

On the other hand, if it is determined that the predetermined condition is not satisfied (step S104: NO), the terminal device 200 transmits a signal indicating a request for acquisition of biometric information to the wearable terminal 300 (step S110).

When receiving the signal indicating a request for acquisition of biometric information, the wearable terminal 300 acquires biometric information (step S112).

Then, the wearable terminal 300 transmits the acquired biometric information to the terminal device 200 (step S114).

When receiving the biometric information, the terminal device 200 performs biometric authentication, based on the received biometric information (step S116). It is assumed here that the biometric authentication is successful.

Then, the terminal device 200 transmits an unlock request signal to the inhaler device 100 (step S118).

When receiving the unlock request signal, the inhaler device 100 is unlocked (step S120). Thereafter, the inhaler device 100 generates an aerosol in accordance with a user operation.

### (4) Supplementary note

While an example in which the request acquirer 10 is included in the terminal device 200 has been described in the present embodiment, the present invention is not limited to such an example. For example, the request acquirer 10 may be included in the inhaler device 100. In this case, when acquiring an unlock request, the inhaler device 100 transmits information indicating that the unlock request has been acquired to the terminal device 200. The other processes are as described above.

### <2.2. Second embodiment>

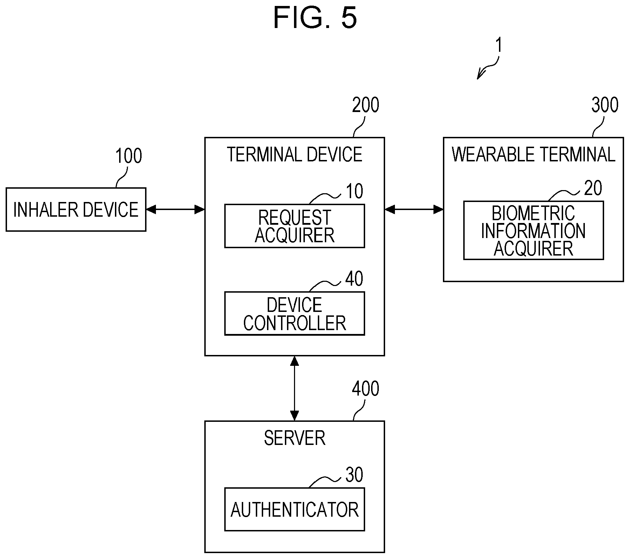

Fig. 5 is a block diagram illustrating a configuration example of a system 1 according to the second embodiment. As illustrated in Fig. 5, the system 1 according to the present embodiment includes an inhaler device 100, a terminal device 200, a wearable terminal 300, and a server 400 as physical structural elements. The inhaler device 100 and the terminal device 200 can communicate with each other, the terminal device 200 and the wearable terminal 300 can communicate with each other, and the terminal device 200 and the server 400 can communicate with each other.

The inhaler device 100, the terminal device 200, and the wearable terminal 300 are as described above. The server 400 is a device located on the Internet. For example, the server 400 may be a so-called cloud server. The present embodiment is different from the first embodiment in that the authenticator 30 is included in the server 400.

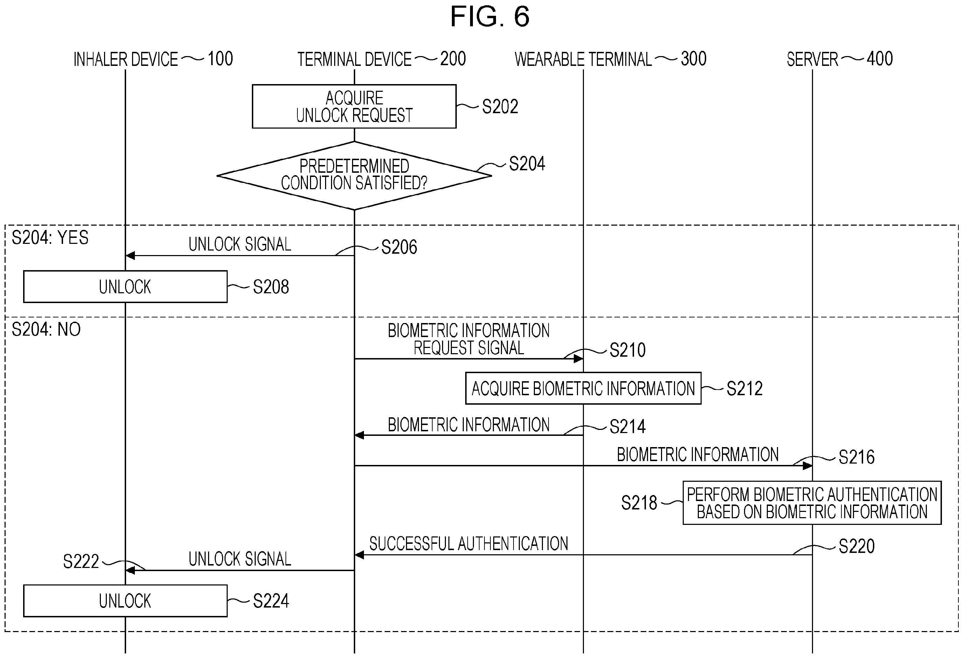

Fig. 6 is a sequence diagram illustrating an example of a process flow executed in the system 1 according to the present embodiment. This sequence involves the inhaler device 100, the terminal device 200, the wearable terminal 300, and the server 400.

As illustrated in Fig. 6, first, the terminal device 200 acquires an unlock request (step S202).

Then, the terminal device 200 determines whether a predetermined condition is satisfied (step S204). For example, the terminal device 200 determines whether an unlock request has been acquired within the first predetermined time after unlocking was performed previously.

If it is determined that the predetermined condition is satisfied (step S204: YES), the terminal device 200 transmits an unlock request signal to the inhaler device 100 (step S206).

When receiving the unlock request signal, the inhaler device 100 is unlocked (step S208). Thereafter, the inhaler device 100 generates an aerosol in accordance with a user operation.

On the other hand, if it is determined that the predetermined condition is not satisfied (step S204: NO), the terminal device 200 transmits a signal indicating a request for acquisition of biometric information to the wearable terminal 300 (step S210).

When receiving the signal indicating a request for acquisition of biometric information, the wearable terminal 300 acquires biometric information (step S212).

Then, the wearable terminal 300 transmits the acquired biometric information to the terminal device 200 (step S214).

When receiving the biometric information, the terminal device 200 transfers the received biometric information to the server 400 (step S216).

When receiving the biometric information, the server 400 performs biometric authentication, based on the received biometric information (step S218). It is assumed here that the biometric authentication is successful.

Then, the server 400 transmits information indicating that the biometric authentication is successful to the terminal device 200 (step S220).

When receiving the information indicating that the biometric authentication is successful, the terminal device 200 transmits an unlock request signal to the inhaler device 100 (step S222).

When receiving the unlock request signal, the inhaler device 100 is unlocked (step S224). Thereafter, the inhaler device 100 generates an aerosol in accordance with a user operation.

In the second embodiment, some or all of the processes executed by the terminal device 200 may be executed by the server 400 by using Progressive Web Apps (PWA). For example, the process in which the terminal device 200 determines whether to perform biometric authentication, based on the received motion information (step S206) may be executed by the server 400 by using the PWA.

### <2.3. Third embodiment>

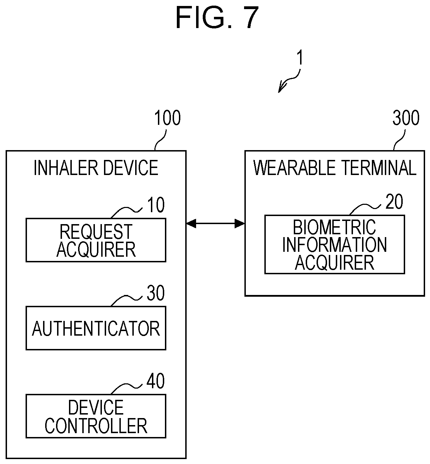

Fig. 7 is a block diagram illustrating a configuration example of a system 1 according to the third embodiment. As illustrated in Fig. 7, the system 1 according to the present embodiment includes an inhaler device 100 and a wearable terminal 300 as physical structural elements. The inhaler device 100 and the wearable terminal 300 can communicate with each other.

The inhaler device 100 and the wearable terminal 300 are as described above. The present embodiment is different from the first embodiment in that the request acquirer 10, the authenticator 30, and the device controller 40 are included in the inhaler device 100.

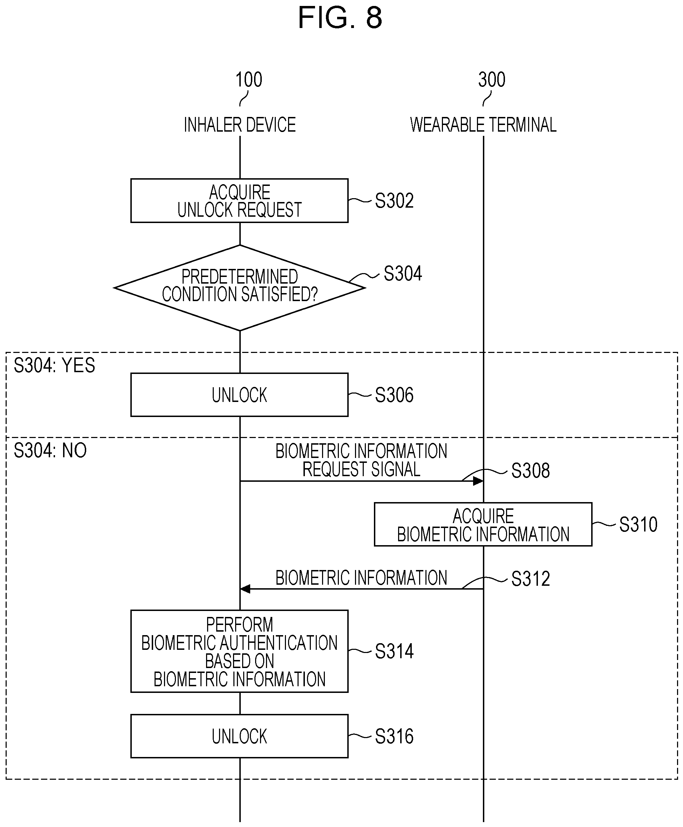

Fig. 8 is a sequence diagram illustrating an example of a process flow executed in the system 1 according to the present embodiment. This sequence involves the inhaler device 100 and the wearable terminal 300.

As illustrated in Fig. 8, first, the inhaler device 100 acquires an unlock request (step S302).

Then, the inhaler device 100 determines whether a predetermined condition is satisfied (step S304). For example, the inhaler device 100 determines whether an unlock request has been acquired within the first predetermined time after unlocking was performed previously.

If it is determined that the predetermined condition is satisfied (step S304: YES), the inhaler device 100 is unlocked (step S306). Thereafter, the inhaler device 100 generates an aerosol in accordance with a user operation.

On the other hand, if it is determined that the predetermined condition is not satisfied (step S304: NO), the inhaler device 100 transmits a signal indicating a request for acquisition of biometric information to the wearable terminal 300 (step S308).

When receiving the signal indicating a request for acquisition of biometric information, the wearable terminal 300 acquires biometric information (step S310).

Then, the wearable terminal 300 transmits the acquired biometric information to the inhaler device 100 (step S312).

When receiving the biometric information, the inhaler device 100 performs biometric authentication, based on the received biometric information (step S314). It is assumed here that the biometric authentication is successful.

Then, the inhaler device 100 is unlocked (step S316). Thereafter, the inhaler device 100 generates an aerosol in accordance with a user operation.

### <2.4. Fourth embodiment>

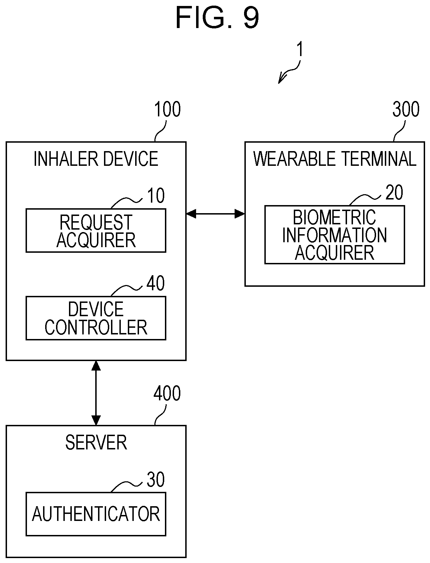

Fig. 9 is a block diagram illustrating a configuration example of a system 1 according to the fourth embodiment. As illustrated in Fig. 9, the system 1 according to the present embodiment includes an inhaler device 100, a wearable terminal 300, and a server 400 as physical structural elements. The inhaler device 100 and the wearable terminal 300 can communicate with each other, and the inhaler device 100 and the server 400 can communicate with each other.

The inhaler device 100, the wearable terminal 300, and the server 400 are as described above. The present embodiment is different from the second embodiment in that the request acquirer 10 and the device controller 40 are included in the inhaler device 100.

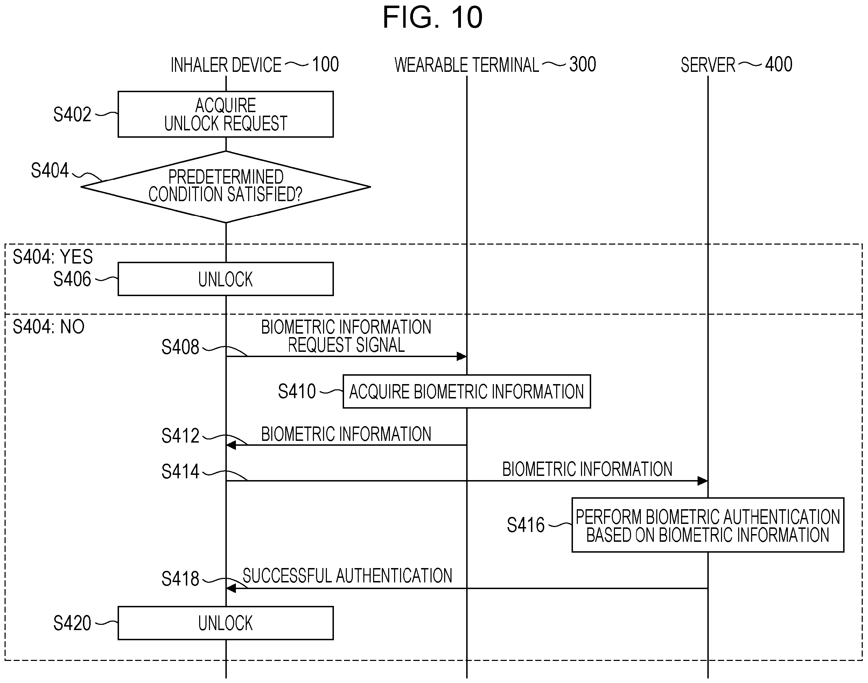

Fig. 10 is a sequence diagram illustrating an example of a process flow executed in the system 1 according to the present embodiment. This sequence involves the inhaler device 100, the wearable terminal 300, and the server 400.

As illustrated in Fig. 10, first, the inhaler device 100 acquires an unlock request (step S402).

Then, the inhaler device 100 determines whether a predetermined condition is satisfied (step S402). For example, the inhaler device 100 determines whether an unlock request has been acquired within the first predetermined time after unlocking was performed previously.

If it is determined that the predetermined condition is satisfied (step S404: YES), the inhaler device 100 is unlocked (step S406). Thereafter, the inhaler device 100 generates an aerosol in accordance with a user operation.

On the other hand, if it is determined that the predetermined condition is not satisfied (step S404: NO), the inhaler device 100 transmits a signal indicating a request for acquisition of biometric information to the wearable terminal 300 (step S408).

When receiving the signal indicating a request for acquisition of biometric information, the wearable terminal 300 acquires biometric information (step S410).

Then, the wearable terminal 300 transmits the acquired biometric information to the inhaler device 100 (step S412).

When receiving the biometric information, the inhaler device 100 transfers the received biometric information to the server 400 (step S414).

When receiving the biometric information, the server 400 performs biometric authentication, based on the received biometric information (step S416). It is assumed here that the biometric authentication is successful.

Then, the server 400 transmits information indicating that the biometric authentication is successful to the inhaler device 100 (step S418).

When receiving the information indicating that the biometric authentication is successful, the inhaler device 100 is unlocked (step S420). Thereafter, the inhaler device 100 generates an aerosol in accordance with a user operation.

While an example in which the authenticator 30 is included in the server 400 has been described in the present embodiment, the authenticator 30 may be included in the terminal device 200 instead of the server 400.

### <3. Hardware configuration example>

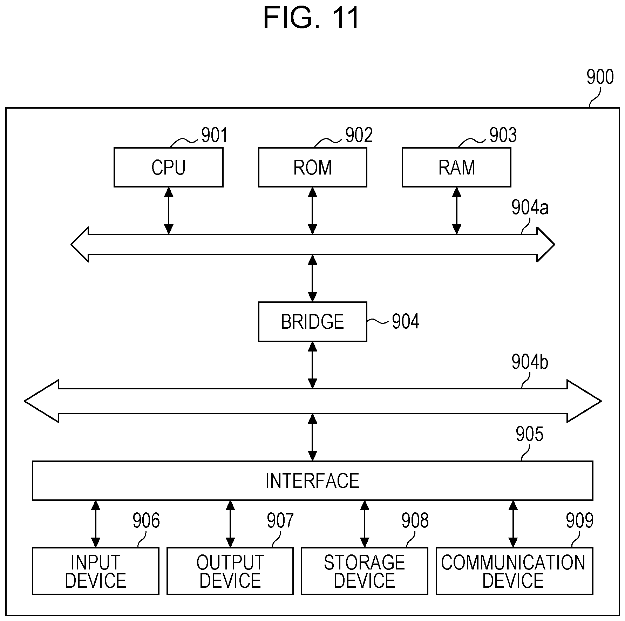

Finally, a hardware configuration of an information processing device according to each of the embodiments described above will be described with reference to Fig. 11. Fig. 11 is a block diagram illustrating an example of a hardware configuration of the information processing device according to the present embodiment. An information processing device 900 illustrated in Fig. 11 may implement, for example, the terminal device 200, the wearable terminal 300, or the server 400 illustrated in Fig. 3, 5, 7, or 9. Information processing performed by the terminal device 200, the wearable terminal 300, or the server 400 according to the present embodiment is implemented by software in cooperation with hardware described hereinafter.

As illustrated in Fig. 11, the information processing device 900 includes a central processing unit (CPU) 901, a read only memory (ROM) 902, a random access memory (RAM) 903, and a host bus 904a. The information processing device 900 further includes a bridge 904, an external bus 904b, an interface 905, an input device 906, an output device 907, a storage device 908, and a communication device 909.

The CPU 901 functions as an arithmetic processing unit and a control circuit, and controls the overall operations of the information processing device 900 in accordance with various programs. The information processing device 900 may include an electric circuit such as a microprocessor, a digital signal processor (DSP), or an application specific integrated circuit (ASIC) instead of or in addition to the CPU 901. The ROM 902 stores programs, operation parameters, and the like used by the CPU 901. The RAM 903 temporarily stores parameters and the like that change as appropriate during execution of a program by the CPU 901. The CPU 901 may form, for example, the authenticator 30 or the device controller 40.

The CPU 901, the ROM 902, and the RAM 903 are connected to each other via the host bus 904a. The host bus 904a is connected to the external bus 904b, such as a Peripheral Component Interconnect/Interface (PCI) bus, via the bridge 904. The host bus 904a, the bridge 904, and the external bus 904b do not necessarily have to be configured separately, and these functions may be implemented in a single bus.

The input device 906 is a device to which information is input from a user. Examples of such a device include a mouse, a keyboard, a touch panel, a button, a switch, and a lever. In addition, the input device 906 may include a microphone that receives a voice input or a camera that receives a gesture input. A user of the information processing device 900 can input various kinds of data to the information processing device 900 or instruct the information processing device 900 to execute processing by operating the input device 906.

In addition, the input device 906 may include a device that detects information on the user. For example, the input device 906 may include various sensors such as a depth sensor (for example, a stereo camera), an acceleration sensor, a gyro sensor, a geomagnetic sensor, an optical sensor, a sound sensor, a biometric sensor, a distance measurement sensor, and a force sensor. Further, the input device 906 may acquire information on the state of the information processing device 900, such as the posture and the movement speed of the information processing device 900, and information on the surrounding environment of the information processing device 900, such as the ambient brightness and noise of the information processing device 900. The input device 906 may further include a global navigation satellite system (GNSS) module that receives a GNSS signal from a GNSS satellite (for example, a global positioning system (GPS) signal from a GPS satellite) and measures position information including latitude, longitude, and altitude of the device. Regarding the position information, the input device 906 may transmit and receive a wireless signal to and from another device, and detect a relative position with respect to the other device. The input device 906 may form, for example, the request acquirer 10 or the biometric information acquirer 20.

The output device 907 is a device that outputs information to a user. Examples of such a device include devices that output visual information, such as a display and a projector, devices that output auditory information, such as a speaker, and devices that output tactile information, such as an eccentric motor. For example, the output device 907 outputs a result obtained by various processes performed by the information processing device 900. In each of the embodiments described above, the output device 907 may output information indicating that an unlock request has been acquired by the request acquirer 10 or the biometric information acquired by the biometric information acquirer 20. In addition, the output device 907 may output information indicating the progress or result of a process performed by the authenticator 30 or the device controller 40.

The storage device 908 is a device that stores data. Examples of such a device include a magnetic storage device such as an HDD, a semiconductor storage device, an optical storage device, and a magneto-optical storage device. The storage device 908 may include a storage medium, a recording device that records data in the storage medium, a reading device that reads data from the storage medium, and a deletion device that deletes data recorded in the storage medium. For example, the storage device 908 may store the biometric information acquired by the biometric information acquirer 20.

The communication device 909 is a device that communicates with another device in a wired or wireless manner. The communication device 909 performs communication conforming to any communication standard such as Wi-Fi (registered trademark), Bluetooth (registered trademark), Long Term Evolution (LTE), low-power wide-area (LPWA), near-field communication (NFC), or universal serial bus (USB). The communication device 909 communicates with the inhaler device 100, the terminal device 200, the wearable terminal 300, or the server 400.

An example of a hardware configuration capable of implementing the functions of the information processing device 900 according to the present embodiment has been described above. Each of the structural elements described above may be implemented using a general-purpose member or may be implemented by hardware specific to the function of each structural element. Thus, the hardware configuration to be used can be changed as appropriate in accordance with the technical level at the time of implementation of the present embodiment.

### <4. Supplementary note>

While preferred embodiments of the present invention have been described in detail with reference to the accompanying drawings, the present invention is not limited to such examples. It will be apparent that those skilled in the art to which the present invention belongs can achieve various modifications or variations without departing from the scope of the technical concept presented in the claims, and it is understood that such modifications or variations also fall within the technical scope of the present invention.

For example, which of the inhaler device 100, the terminal device 200, the wearable terminal 300, and the server 400 includes each of the request acquirer 10, the biometric information acquirer 20, the authenticator 30, and the device controller 40 is not limited to that in each of the embodiments described above. That is, each of the request acquirer 10, the biometric information acquirer 20, the authenticator 30, and the device controller 40 may be mounted on any one of the inhaler device 100, the terminal device 200, the wearable terminal 300, and the server 400. For example, the biometric information acquirer 20 may be included in the inhaler device 100 or the terminal device 200.

For example, in the embodiments described above, the predetermined condition is a condition that the inhaler device 100 has been unlocked within the first predetermined time before an unlock request is acquired. However, the present invention is not limited to such an example. The predetermined condition may include, in addition to or instead of the above condition, a condition that biometric authentication based on biometric information acquired within a second predetermined time before an unlock request is acquired has succeeded. That is, if an unlock request is acquired within the second predetermined time after biometric authentication is successful, the device controller 40 may determine to perform unlocking without performing biometric authentication again. This configuration allows biometric authentication to be performed again if the second predetermined time has elapsed since the biometric authentication was performed previously, making it possible to improve usability while maintaining security, as in the embodiments described above. The second predetermined time may also be set in accordance with at least one of the position of the user, the situation around the user, or the time period.

For example, in the embodiments described above, unlocking has been described as an example of the predetermined process. However, the present invention is not limited to such an example. The predetermined process may be any process executed by the inhaler device 100. For example, the predetermined process may be transmission of an operation log. The device controller 40 can determine to transmit an operation log without performing biometric authentication if a request for transmitting the operation log is acquired within the first predetermined time after the operation log was transmitted previously, and to transmit an operation log after performing biometric authentication otherwise.

A series of processes performed by each of the devices described herein may be implemented using any one of software, hardware, and a combination of software and hardware. A program constituting the software is stored in advance in, for example, an internal or external recording medium (non-transitory medium) of each device. For example, each program is read into a RAM at the time of execution by a computer that controls each of the devices described herein, and is executed by a processor such as a CPU. Examples of the recording medium include a magnetic disk, an optical disk, a magneto-optical disk, and a flash memory. Further, the computer program described above may be distributed via, for example, a network without using a recording medium.

In addition, some or all of the processes executed by the terminal device 200 may be executed by the server 400 or any other server (not illustrated) by using Progressive Web Apps (PWA).

The processes described herein using the flowcharts and the sequence diagrams need not be executed in the illustrated order. Some processing steps may be executed in parallel. Any additional processing step may be used, or some processing steps may be omitted.

The following configurations also fall within the technical scope of the present invention.
(1) An information processing method including:
   acquiring a predetermined request for requesting an inhaler device to execute a predetermined process, the inhaler device being configured to generate an aerosol to be inhaled by a user; and
   determining whether to perform biometric authentication based on biometric information of the user before the predetermined process is executed, in accordance with whether a predetermined condition is satisfied.
(2) The information processing method according to (1), wherein
   the determining of whether to perform biometric authentication includes:
   determining not to perform the biometric authentication before the predetermined process is executed, when the predetermined condition is satisfied; and
   determining to perform the biometric authentication before the predetermined process is executed, when the predetermined condition is not satisfied.
(3) The information processing method according to (2), further including:
   controlling the inhaler device to execute the predetermined process when the predetermined condition is satisfied.
(4) The information processing method according to (2) or (3), further including:
   when the predetermined condition is not satisfied,
   acquiring the biometric information of the user;
   performing the biometric authentication, based on the acquired biometric information; and
   controlling the inhaler device to execute the predetermined process in response to the biometric authentication being successful.
(5) The information processing method according to any one of (1) to (4), wherein
   the predetermined condition includes a condition that the inhaler device has executed the predetermined process within a first predetermined time before the predetermined request is acquired.
(6) The information processing method according to (5), wherein
   the first predetermined time is set in accordance with a position of the user.
(7) The information processing method according to (5) or (6), wherein
   the first predetermined time is set in accordance with a situation around the user.
(8)The information processing method according to any one of (5) to (7), wherein
   the first predetermined time is set in accordance with a time period.
(9) The information processing method according to any one of (1) to (8), wherein
   the predetermined condition includes a condition that the biometric authentication based on the biometric information acquired within a second predetermined time before the predetermined request is acquired has succeeded.
(10) The information processing method according to any one of (1) to (9), wherein
   the predetermined process is a process of permitting execution of a process of generating the aerosol.
(11) The information processing method according to any one of (1) to (10), wherein
   the biometric authentication is performed based on a plurality of types of the biometric information.
(12) The information processing method according to any one of (1) to (11), wherein
   the predetermined request is acquired by the inhaler device.
(13) The information processing method according to any one of (1) to (11), wherein
   the predetermined request is acquired by a terminal device associated with the inhaler device in advance.
(14) The information processing method according to any one of (1) to (13), wherein
   the biometric information is acquired by a wearable terminal worn by the user.
(15) The information processing method according to any one of (1) to (14), wherein
   the biometric authentication is performed by the inhaler device.
(16) The information processing method according to any one of (1) to (14), wherein
   the biometric authentication is performed by a terminal device associated with the inhaler device in advance.
(17) The information processing method according to any one of (1) to (14), wherein
   the biometric authentication is performed by a server located on the Internet.
(18) An aerosol generation system including:
   a request acquirer configured to acquire a predetermined request for requesting an inhaler device to execute a predetermined process, the inhaler device being configured to generate an aerosol to be inhaled by a user; and
   a device controller configured to determine whether to perform biometric authentication based on biometric information of the user before the predetermined process is executed, in accordance with whether a predetermined condition is satisfied.
(19) The aerosol generation system according to (18), wherein
   the inhaler device is configured to generate the aerosol by using a substrate including at least one of an aerosol source from which the aerosol is generated or a flavor source from which a flavor to be imparted to the aerosol is generated, and
   the aerosol generation system includes the substrate.
(20) A program for causing a computer to execute:
   in response to acquisition of a predetermined request for requesting an inhaler device to execute a predetermined process, the inhaler device being configured to generate an aerosol to be inhaled by a user, determining whether to perform biometric authentication based on biometric information of the user before the predetermined process is executed, in accordance with whether a predetermined condition is satisfied.

### Reference Signs List

- 1: system
- 100: inhaler device
- 110: power supply unit
- 111: power supply
- 112: sensor
- 113: notifier
- 114: memory
- 115: communicator
- 116: controller
- 120: cartridge
- 121: heater
- 122: liquid guide
- 123: liquid storage
- 124: mouthpiece
- 130: flavor imparting cartridge
- 140: container
- 141: internal space
- 142: opening
- 143: bottom
- 150: stick substrate
- 151: substrate
- 152: inhalation port
- 180: airflow path
- 181: air inlet hole
- 182: air outlet hole
- 200: terminal device
- 300: wearable terminal
- 400: server
- 10: request acquirer
- 20: biometric information acquirer
- 30: authenticator
- 40: device controller

## Claims

1. An information processing method comprising:
acquiring a predetermined request for requesting an inhaler device to execute a predetermined process, the inhaler device being configured to generate an aerosol to be inhaled by a user; and
determining whether to perform biometric authentication based on biometric information of the user before the predetermined process is executed, in accordance with whether a predetermined condition is satisfied.

2. The information processing method according to claim 1, wherein
the determining of whether to perform biometric authentication includes:
determining not to perform the biometric authentication before the predetermined process is executed, when the predetermined condition is satisfied; and
determining to perform the biometric authentication before the predetermined process is executed, when the predetermined condition is not satisfied.

3. The information processing method according to claim 2, further comprising:
controlling the inhaler device to execute the predetermined process when the predetermined condition is satisfied.

4. The information processing method according to claim 2 or 3, further comprising:
when the predetermined condition is not satisfied,
acquiring the biometric information of the user;
performing the biometric authentication, based on the acquired biometric information; and
controlling the inhaler device to execute the predetermined process in response to the biometric authentication being successful.

5. The information processing method according to any one of claims 1 to 4, wherein
the predetermined condition includes a condition that the inhaler device has executed the predetermined process within a first predetermined time before the predetermined request is acquired.

6. The information processing method according to claim 5, wherein
the first predetermined time is set in accordance with a position of the user.

7. The information processing method according to claim 5 or 6, wherein
the first predetermined time is set in accordance with a situation around the user.

8. The information processing method according to any one of claims 5 to 7, wherein
the first predetermined time is set in accordance with a time period.

9. The information processing method according to any one of claims 1 to 8, wherein
the predetermined condition includes a condition that the biometric authentication based on the biometric information acquired within a second predetermined time before the predetermined request is acquired has succeeded.

10. The information processing method according to any one of claims 1 to 9, wherein
the predetermined process is a process of permitting execution of a process of generating the aerosol.

11. The information processing method according to any one of claims 1 to 10, wherein
the biometric authentication is performed based on a plurality of types of the biometric information.

12. The information processing method according to any one of claims 1 to 11, wherein
the predetermined request is acquired by the inhaler device.

13. The information processing method according to any one of claims 1 to 11, wherein
the predetermined request is acquired by a terminal device associated with the inhaler device in advance.

14. The information processing method according to any one of claims 1 to 13, wherein
the biometric information is acquired by a wearable terminal worn by the user.

15. The information processing method according to any one of claims 1 to 14, wherein
the biometric authentication is performed by the inhaler device.

16. The information processing method according to any one of claims 1 to 14, wherein
the biometric authentication is performed by a terminal device associated with the inhaler device in advance.

17. The information processing method according to any one of claims 1 to 14, wherein
the biometric authentication is performed by a server located on the Internet.

18. An aerosol generation system comprising:
a request acquirer configured to acquire a predetermined request for requesting an inhaler device to execute a predetermined process, the inhaler device being configured to generate an aerosol to be inhaled by a user; and
a device controller configured to determine whether to perform biometric authentication based on biometric information of the user before the predetermined process is executed, in accordance with whether a predetermined condition is satisfied.

19. The aerosol generation system according to claim 18, wherein
the inhaler device is configured to generate the aerosol by using a substrate including at least one of an aerosol source from which the aerosol is generated or a flavor source from which a flavor to be imparted to the aerosol is generated, and
the aerosol generation system includes the substrate.

20. A program for causing a computer to execute:
in response to acquisition of a predetermined request for requesting an inhaler device to execute a predetermined process, the inhaler device being configured to generate an aerosol to be inhaled by a user, determining whether to perform biometric authentication based on biometric information of the user before the predetermined process is executed, in accordance with whether a predetermined condition is satisfied.
